# DEMANDE DE BREVET EUROPEEN

(11) **EP 4 640 640 A1**
(43) Date de publication de la demande: **29.10.2025**
(21) Numéro de dépôt: 25172223.7
(22) Date de dépôt: 24.04.2025
(51) Int. Cl.: C02F 3/34, C02F 101/16, C02F 103/42, C12N 1/20, C12R 1/125

(54) **COMPOSITION BACTÉRIENNE POUR LE TRAITEMENT BIOLOGIQUE D'UN SYSTÈME AQUATIQUE**

(30) Priorité: 24.04.2024 BE 202405242
(71) Demandeur: Aquatic Science SA, 4040 Herstal (BE); VITII, 4040 Herstal (BE)
(72) Inventeur: LUIZI, Frédéric, 4040 Herstal (BE)
(74) Mandataire: Calysta NV

(57) **Abrégé**

Composition pour le traitement biologique des systèmes aquatiques, comprenant un agent actif bactérien, ledit agent actif bactérien comprenant des bactéries du genre *Bacillus* et *Paracoccus*, caractérisée en ce que la quantité des bactéries du genre *Bacillus* et *Paracoccus* dans ledit agent actif bactérien est supérieure ou égale à 80% en poids par rapport au poids total dudit agent actif bactérien dans la composition.

## Description

### Domaine technique

La présente invention vise une composition adaptée pour optimiser un traitement biologique des systèmes aquatiques. Plus spécifiquement, elle vise une composition comprenant un agent actif bactérien comprenant des bactéries du genre *Bacillus* et Paracoccus permettant un bon accomplissement du cycle de l'azote.

La présente invention se rapporte également à l'utilisation de la composition pour traiter un système aquatique, telles que des bassins d'eaux récréatives, des piscines biologiques/naturelles, spas, jacuzzis, bassins à poissons, mares décoratives et parcs aquatiques.

La présente invention se rapporte aussi à un procédé de préparation de la composition selon la présente invention.

### Art antérieur

Les eaux, comme par exemple les eaux à usage récréatif, nécessitent d'être traitées pour éviter la dégradation de leur qualité ainsi que la prolifération de bactéries et d'algues.

Un traitement chimique des eaux récréatives, comme par exemple un traitement au chlore, est connu depuis de nombreuses années.

Le traitement au chlore permet une réduction efficace de la présence d'algues, de microorganismes et assure une bonne purification de l'eau. Cependant, la désinfection au chlore peut amener différents effets nocifs pour l'être humain, en fonction de la dose de chlore et du temps d'exposition, tels que le dessèchement de l'épiderme, une gêne respiratoire, une irritation des muqueuses, etc. D'autre part, si le traitement au chlore n'est pas opérationnel pendant un certain temps dans un système d'eaux récréatives, un développement rapide de bactéries peut avoir lieu. Ceci peut être très nocif pour les utilisateurs et engendre également une détérioration visuelle de l'aspect des eaux qui deviennent troubles et changent de couleur, ce qui génère une impression négative pour les utilisateurs.

Une alternative courante à la chloration est l'élimination biologique des composés azotés inorganiques, tels que les ions ammonium (NH₄⁺) et nitrate (NO₃⁻) des systèmes aquatiques.

Des solutions pour traiter biologiquement des systèmes aquatiques ou des eaux usées existent déjà.

Dans ce contexte, le document WO 2016/179 390 décrit une composition et une méthode pour éliminer le nitrate d'un milieu aqueux dans des conditions aérobies comprenant des bactéries. Les bactéries décrites comprennent entre autres des souches de Bacillus. Ce document présente l'inconvénient de nécessiter des niveaux élevés d'oxygène dissous. La méthode se concentre sur la dégradation des nitrates mais ne prend pas en compte la dégradation de la matière organique ou de tout autre polluant. De plus, les consommateurs indiquent que la méthode selon ce document nécessite toujours une chloration supplémentaire.

Le document EP2178802 concerne le traitement de l'eau et des procédés de contrôle du sulfure d'hydrogène dans des environnements aquatiques et marins. Ce document décrit un procédé pour éliminer le sulfure d'hydrogène d'un système aquatique de manière à augmenter le rendement de production d'animaux aquatiques, comme par exemple la production de crevettes. Le procédé selon ce document utilise une composition comprenant une quantité suffisante de bactéries du genre Paracoccus pour contrôler, réduire ou éliminer le sulfure d'hydrogène dans le système aquatique.

Le document EP3712116 concerne une composition pour le traitement biologique des piscines. Ce document décrit une composition comprenant un mélange d'espèces sélectionnées parmi les genres Bacillus, *Paenibacillus* et Paracoccus pour contrôler le cycle de l'azote. Ce document mentionne que la plus grande réduction globale de la concentration en phosphore et en nitrite a été observée pour une composition comprenant un « cocktail » combinant une pluralité de souches de Bacillus, *Paenibacillus* et Paracoccus, en particulier un cocktail bactérien comprenant *Bacillus circulans, Bacillus lichenformis, Bacillus amyloliquefaciens, Bacillus pumilis, Bacillus subtilis, Phaenibacillus polymixa,* et Paracoccus sp. En pratique, bien que la composition selon ce document soit utile pour traiter biologiquement des systèmes aquatiques et en particulier pour accomplir le cycle de l'azote, cette composition présente beaucoup d'inconvénients. Notamment, elle est difficilement industrialisable et fastidieuse (donc couteuse) : elle nécessite la préparation d'un mélange de nombreuses bactéries, qui sont communément produites/fermentées séparément. De plus, elle demande un équilibre subtil et fragile entre les différentes populations bactériennes, pour garder l'efficacité du traitement biologique. En effet, si l'équilibre entre les différentes populations bactériennes n'est pas adéquat, une population bactérienne de la composition peut devenir prépondérante sur les autres, ce qui empêchera d'accomplir correctement le cycle de l'azote et de réaliser un traitement biologique efficace.

Il existe donc toujours un besoin d'avoir une composition facilement industrialisable et stable, tout en permettant de réaliser un traitement biologique de systèmes aquatiques de manière efficace (en assurant par exemple un bon accomplissement du cycle de l'azote).

### Objectifs de l'invention

La présente invention a pour but de pallier les inconvénients de l'état de la technique, notamment ceux décrits ci-dessus.

En particulier, la présente invention se propose de fournir une composition simple et facilement industrialisable pour traiter biologiquement des systèmes aquatiques.

La présente invention se propose également de fournir une composition stable permettant d'accomplir de manière efficace l'ensemble du cycle de l'azote.

### Résumé de l'invention

Pour atteindre les objectifs précités, il est prévu selon l'invention une composition pour le traitement biologique des systèmes aquatiques, comprenant un agent actif bactérien, l'agent actif bactérien comprenant des bactéries du genre *Bacillus* et Paracoccus, caractérisée en ce que la quantité des bactéries du genre *Bacillus* et Paracoccus dans l'agent actif bactérien est supérieure ou égale à 80% en poids par rapport au poids total dudit agent actif bactérien dans la composition.

Les inventeurs ont en effet découvert, de manière surprenante qu'une composition pour le traitement biologique des systèmes aquatiques avec un agent actif bactérien simplifié comprenant en grande majorité spécifiquement *Bacillus* et Paracoccus permet d'avoir une excellente efficacité, notamment même équivalente voire supérieure à des mélanges complexes de l'état de la technique, mais en permettant une simplification très importante de la formulation bactérienne.

La composition de l'invention est donc très facilement industrialisable car elle ne nécessite que la production de deux types de bactéries principales. De plus, de manière particulièrement étonnante, avec un agent actif bactérien « simple », cette composition est particulièrement efficace pour réaliser toutes les étapes du cycle de l'azote, à savoir la décomposition de la matière organique par des hétérotrophes pour former des composés ammoniacaux, la nitrosation des composés ammoniacaux pour former des nitrites, la nitratation des nitrites pour former des nitrates et la dénitrification du nitrate pour former de l'azote gazeux. De plus, comme mentionné, cette composition est particulièrement stable pour assurer l'accomplissement du cycle de l'azote car un équilibre adéquat est rapidement atteint et maintenu entre *Bacillus* et Paracoccus, en comparaison avec le cas d'un mélange complexe de nombreuses bactéries différentes dont l'équilibre est délicat à obtenir et nettement plus instable à maintenir.

D'autres formes de réalisation de la composition pour le traitement biologique des systèmes aquatiques selon la présente invention sont indiquées dans les revendications annexées.

La présente invention se rapporte également à l'utilisation de la composition selon la présente invention pour traiter biologiquement un système aquatique telles que des bassins d'eaux récréatives, des piscines biologiques/naturelles, spas, jacuzzis, bassins à poissons, aquariums, mares décoratives et parcs aquatiques.

Par l'expression « des bassins d'eaux récréatives », il est fait référence à tout plan d'eau utilisé à des fins de loisirs, y compris, sans s'y limiter, les piscines, les lagunes artificielles, les rivières lentes et autres installations aquatiques destinées à la baignade, à la détente ou à d'autre activités aquatiques.

D'autres formes de réalisation l'utilisation de la composition pour le traitement biologique des systèmes aquatiques selon la présente invention sont indiquées dans les revendications annexées.

La présente invention se rapporte aussi à un procédé de préparation de la composition selon la présente invention, dans lequel chacune des bactéries de l'agent actif bactérien est fermentée, optionnellement séchée et récoltée individuellement.

Par l'expression « chacune des bactéries de l'agent actif bactérien », il est bien évidemment entendu dans le domaine de l'invention, et serait compris par l'homme du métier, qu'il s'agit de chacune des différentes espèces/genres de bactéries de l'agent actif bactérien qui sont fermentées, optionnellement séchées et récoltée individuellement, et non pas chaque bactérie prise individuellement.

D'autres formes de réalisation du procédé de préparation de la composition pour le traitement biologique des systèmes aquatiques selon la présente invention sont indiquées dans les revendications annexées.

La présente invention se rapporte aussi à un filtre biologique pour des bassins d'eaux récréatives comprenant, en mode opérationnel, un substrat colonisé par les bactéries de l'agent actif bactérien de la composition selon la présente invention.

Plus particulièrement, le filtre biologique pour des bassins d'eaux récréatives selon l'invention comprend, en mode opérationnel, un substrat colonisé par les bactéries de l'agent actif bactérien de la composition pour le traitement biologique des systèmes aquatiques dans laquelle l'agent actif bactérien comprend des bactéries du genre *Bacillus* et Paracoccus, la quantité des bactéries du genre *Bacillus* et Paracoccus dans l'agent actif bactérien étant supérieure ou égale à 80% en poids par rapport au poids total dudit agent actif bactérien dans la composition.

D'autres formes de réalisation du filtre biologique selon la présente invention sont indiquées dans les revendications annexées.

La présente invention se rapporte également à un système aquatique comprenant le filtre biologique selon la présente invention.

D'autres formes de réalisation du système aquatique selon la présente invention sont indiquées dans les revendications annexées.

### Description détaillée de l'invention

D'autres caractéristiques et avantages de la présente invention seront dérivés de la description suivante non limitative, et en faisant référence aux figures suivantes :
La figure 1 montre l'évolution temporelle de la concentration de différents paramètres biologiques d'une eau traitée par une composition selon l'invention.
La figure 2 montre l'évolution temporelle de la concentration de différents paramètres biologiques d'une eau traitée par une première composition comparative.
La figure 3 montre l'évolution temporelle de la concentration de différents paramètres biologiques d'une eau traitée par une deuxième composition comparative.
La figure 4 montre l'évolution temporelle de la concentration de différents paramètres biologiques d'une eau traitée par une troisième composition comparative.
La figure 5 montre l'évolution temporelle de la concentration de différents paramètres biologiques d'une eau d'une piscine à grande échelle traitée par une composition selon la présente invention et par la première composition comparative.

Dans la présente description et les revendications, il est bien entendu que les termes « un », « une » ou « le » signifient « au moins un » et ne doivent pas être limités à « un seul », sauf indication contraire explicite. De plus, lorsqu'une plage de valeur est indiquée, les extrémités sont incluses. Finalement, toutes les valeurs intégrales et de sous-domaine dans une gamme/plage numérique sont expressément incluses comme si explicitement écrites.

Dans le contexte de la présente invention, on entend par le terme « système aquatique », un bassin d'eau comme par exemple des piscines biologiques/naturelles, des spas, des jacuzzis, des bassins de baignage, et/ou des bassins à poissons, des mares décoratives, des parcs aquatiques, des bassins de baignade lacustres, etc.

Dans le contexte de la présente invention, le terme « un agent actif bactérien » inclut les bactéries de la composition qui sont actives dans le traitement biologique d'une eau ou, en d'autres mots, qui sont aptes à réaliser un traitement biologique d'une eau, et en particulier à réaliser une ou plusieurs étapes du cycle de l'azote. Par soucis de clarté, cela signifie dès lors que, si la composition comprend des bactéries qui ne sont pas aptes à réaliser un traitement biologique d'une eau, et en particulier apte à réaliser une ou plusieurs étapes du cycle de l'azote, elles ne sont pas comprises dans l'agent actif bactérien selon l'invention.

De préférence, dans la composition de l'invention, la quantité des bactéries du genre *Bacillus* et Paracoccus dans l'agent actif bactérien est supérieure ou égale à 85%, préférentiellement 90%, plus préférentiellement 95%, 96%, 97%, 98%, voire 99% en poids par rapport au poids total de l'agent actif bactérien dans la composition.

De manière avantageuse, l'agent actif bactérien consiste en des bactéries du genre *Bacillus* et Paracoccus. En effet, plus l'agent actif bactérien présentera une proportion élevée en poids des bactéries du genre Bacillus et Paracoccus par rapport au poids total de l'agent actif bactérien, plus la composition selon l'invention sera basée sur un petit nombre de bactéries différentes actives dans le traitement biologique des eaux, tout en restant très efficace. Ceci est particulièrement avantageux, notamment, de manière à pouvoir réaliser une industrialisation facile de la composition selon l'invention.

De préférence, dans la composition de l'invention, les bactéries du genre *Bacillus* et Paracoccus présente un rapport en poids Bacillus/Paracoccus compris entre 1:10 et 10:1, préférentiellement compris entre 1:5 et 5:1, de manière avantageuse d'environ 1:1. En effet, ce rapport en poids Bacillus/Paracoccus s'est avéré particulièrement efficace pour accomplir de manière optimale et rapide les différentes étapes du cycle de l'azote.

De préférence, les bactéries du genre *Bacillus* sont choisies dans le groupe constitué de *Bacillus pumilus, Bacillus licheniformis, Bacillus amyloliquefaciens, Bacillus circulans et Bacillus subtilis,* de préférence *Bacillus subtilis.* De préférence, *Bacillus subtilis* a un poids égal ou supérieur à 80%, de manière plus préférée 85%, 90%, 95%, 96%, 97%, 98%, voire 99% du poids total des bactéries du genre Bacillus. En effet, *Bacillus subtilis* est très facilement manipulable assurant une industrialisation aisée de la composition selon l'invention. De plus, dans la composition de l'invention et plus spécifiquement en combinaison avec des bactéries du genre Paracoccus, *Bacillus subtilis* s'est montrée particulièrement efficace pour assurer la dégradation de la matière organique et/ou pour assurer l'étape de dénitrification du cycle de l'azote.

De préférence, les bactéries du genre Paracoccus sont choisies dans le groupe constitué de Paracoccus *denitrificans,* Paracoccus *ferrooxidans*, Paracoccus *haeundaensis,* Paracoccus halotolerans, Paracoccus *homiensis,* Paracoccus *kawasakiensis, Paracoccus kocurii,* Paracoccus kondratievae, Paracoccus *koreensis, Paracoccus marcusii, Paracoccus* methylutens, Paracoccus pantotrophus, Paracoccus *seriniphilus, Paracoccus solvantivorans, Paracoccus alcaliphilus, Paracoccus alkenifer, Paracoccus aminophilus,* Paracoccus aminovorans, Paracoccus *bengalensis, Paracoccus carotinifaciens,* Paracoccus *thiocyanatus, Paracoccus thiophilus, Paracoccus versutus,* Paracoccus yeei et Paracoccus *zeaxanthinifaciens,* de préférence Paracoccus *denitrificans,* Paracoccus aminovorans et Paracoccus pantotrophus, De préférence Paracoccus pantotrophus. De préférence, les bactéries du genre Paracoccus sont Paracoccus pantotrophus. Selon ce mode de réalisation, Paracoccus pantotrophus a un poids égal ou supérieur à 80%, de manière plus préférée 85%, 90%, 95%, 96%, 97%, 98%, voire 99% du poids total des bactéries du genre Paracoccus dans la composition. En effet, dans la composition de l'invention et plus spécifiquement en combinaison avec des bactéries du genre Bacillus, *Paracoccus* pantotrophus montre une grande complémentarité avec *Bacillus* pour accomplir les étapes du cycle de l'azote car elle permet non seulement de transformer la matière organique en ions ammonium et en ammoniaque mais aussi de transformer ces ions et l'ammoniaque en nitrite et puis en nitrate.

De préférence, l'agent actif bactérien comprend *Bacillus subtilis* et Paracoccus pantotrophus, et la quantité de *Bacillus subtilis* et Paracoccus pantotrophus dans l'agent actif bactérien est supérieure ou égale à 80%, de manière favorable supérieur ou égale à 85%, 90%, 95%, 96%, 97%, 98%, voire 99% en poids par rapport au poids total de l'agent actif bactérien dans la composition selon l'invention. De préférence, dans l'agent actif bactérien, les bactéries *Bacillus subtilis* et Paracoccus pantotrophus présente un rapport en poids *Bacillus subtilis*/*Paracoccus* pantotrophus compris entre 1:10 et 10:1, préférentiellement compris entre 1:5 et 5:1, de manière avantageuse d'environ 1:1. De manière plus préférée, l'agent actif bactérien comprend *Bacillus subtilis* et Paracoccus pantotrophus, et la quantité de *Bacillus subtilis* et Paracoccus pantotrophus dans l'agent actif bactérien est supérieure ou égale à 80%, 90%, 95%, 96%, 97%, 98%, voire 99% en poids par rapport au poids total de l'agent actif bactérien dans la composition selon l'invention, et les bactéries *Bacillus subtilis* et Paracoccus pantotrophus de l'agent actif bactérien présente un rapport en poids *Bacillus subtilis*/*Paracoccus* pantotrophus compris entre 1:5 et 5:1, de manière avantageuse d'environ 1:1.

De préférence, la composition selon l'invention comprend en outre un agent enzymatique. En effet, un agent enzymatique permet d'avoir une action bactériostatique rapide sur certains types de bactéries pouvant améliorer la prolifération et l'établissement de l'agent actif bactérien de manière à optimiser le traitement biologique des eaux. De plus, une composition comprenant un agent enzymatique apporte une synergie et élimine efficacement les polluants de la piscine. Les enzymes dissocient les molécules polluantes en formes plus simples et les bactéries utilisent ces intermédiaires simples dans leurs activités métaboliques, dégradant ainsi complètement les polluants présents dans les eaux usées. Les microbes se développent plus rapidement en raison de la disponibilité croissante d'intermédiaires simples et produisent davantage d'enzymes susceptibles de dégrader davantage les polluants. L'ajout de l'agent enzymatique est particulièrement pertinent lorsque la composition est ajoutée dans le système aquatique pour la première fois. La dissociation enzymatique des molécules polluantes facilite ainsi la colonisation initiale du système aquatique.

De préférence, l'agent enzymatique comprend une ou plusieurs enzymes choisie(s) dans le groupe constitué de : peroxidases, lignine peroxidases, laccases, catalases, cytochrome c-oxidases, glucose oxidases, phenol oxidases, n- and o-demethylases, proteases, lipases, alpha-amylases, bacteriocins, papaya peptidase. De manière favorable, l'agent enzymatique comprend papaya peptidase. En effet cette enzyme est active à une très grande gamme de températures, ce qui la rend particulièrement adaptée dans la composition selon l'invention pour traiter biologiquement des systèmes aquatiques.

De préférence, l'agent enzymatique et l'agent actif bactérien dans la composition présente un rapport pondéral (agent enzymatique/agent actif bactérien) compris entre 1 :10000 et 1000 :1, de préférence entre 1 :1000 et 1 :1.

De manière préférée, la composition selon l'invention comprend en outre un agent minéral. En effet, un agent minéral fournit un support de croissance et/ou une source de nutriments favorisant la croissance de l'agent actif bactérien de la composition selon l'invention.

De préférence, l'agent minéral comprend un ou plusieurs macroéléments sélectionnés dans le groupe : sulfate de magnésium, chlorure de sodium, carbonate de sodium, bicarbonate de sodium, chlorure de calcium, chlorure de magnésium, magnésium sulfate et sulfate de potassium, de préférence le carbonate de sodium, le bicarbonate de sodium, le chlorure de calcium et le sulfate de magnésium.

De préférence, l'agent minéral comprend un ou plusieurs oligoéléments sélectionnés parmi le cuivre, cobalt, chrome, molybdène, nickel, tungstène, et zinc.

De manière favorable, la composition selon l'invention comprend un agent enzymatique et un agent minéral. En effet, l'agent minéral comprenant des sels inorganiques d'ions métalliques, est très utile à la catalyse enzymatique facilitant la dissociation des liaisons de la molécule polluante, ce qui est important car plus tôt la dissociation des liaisons est précoce, plus tôt est la dégradation des polluants.

De préférence, la composition est sous forme d'une poudre, d'une suspension aqueuse, d'une émulsion aqueuse ou d'une combinaison de celles-ci, de préférence sous forme d'une suspension aqueuse et/ou d'une émulsion aqueuse.

De préférence, la composition selon l'invention comprend en outre un agent de conservation. De préférence, l'agent de conservation a un poids compris entre 10 et 50 %, de préférence entre 20 et 40 %, de manière favorable entre 25 et 35 % du poids total de la composition. Pour une conservation à long terme, les bactéries sont généralement lyophilisées ou séchées par pulvérisation. Pour réactiver des bactéries ainsi traitées, il faut compter quelques jours à quelques semaines selon les souches. Durant cette période, l'eau subit d'importantes modifications dues au développement progressif de toutes les souches bactériennes. Comme dans les piscines, par exemple les piscines biologiques, aucun désinfectant ne peut être utilisé pour clarifier l'eau, il peut y avoir une première période pendant laquelle la piscine peut être affectée par une prolifération d'algues. En incorporant un agent de conservation, l'agent bactérien est protégé des dommages lors de la congélation, de la lyophilisation, de la décongélation, du stockage ou de toute autre manipulation. Comme l'agent bactérien est protégé, la période de réactivation est raccourcie et aucune réactivation de l'agent bactérien avant l'ajout à l'eau n'est nécessaire. Les inventeurs ont observé que des concentrations d'agents de conservation d'environ 10 % en poids à 50 % en poids donnaient les meilleurs résultats. De manière plus préférée, l'agent de conservation est un cryoprotecteur, de préférence le propylène glycol, ou de la saccharose. En effet, l'incorporation d'un cryoprotecteur protège spécifiquement les cellules bactériennes des dommages lors de la congélation, de la lyophilisation, de la décongélation ou du stockage.

De préférence, l'utilisation de la composition selon la présente invention pour traiter biologiquement un système aquatique, comprend un ajout dans le système aquatique d'une quantité de la composition comprise entre 4 g et 200 g de la composition par m³ d'eau à traiter du système aquatique. De préférence, l'utilisation comprend plusieurs ajouts à intervalle de temps régulier dans le système aquatique d'une quantité de la composition comprise entre 2 g et 100 g de la composition par m³ d'eau à traiter du système aquatique. De préférence, l'intervalle de temps régulier est compris entre 5 jours et 28 jours.

De préférence, dans le procédé de préparation de la composition selon l'invention, les bactéries sont fermentées de manière aérobie. Optionnellement, pour les bactéries capables de former des spores, le processus de fermentation comprend une étape de « choc » pour amener les bactéries sous forme de spores (sporulation). N'importe quelle méthode de « choc » connue dans la technique convient pour ce procédé. Par exemple, la fermentation peut être soumise à un choc thermique pour obtenir une sporulation.

Dans certains modes de réalisation du procédé de préparation de la composition selon l'invention, les bactéries sont fermentées de manière anaérobie en présence de glucides. Les glucides appropriés comprennent par exemple l'inuline, le fructo-oligosaccharide et les gluco-oligosaccharides.

De plus, en fermentant et en récoltant individuellement les différentes espèces de l'agent bactérien, la composition est facilement ajustée. Ceci permet une plus grande flexibilité lors de la production. De plus, en fermentant chaque espèce séparément, chaque espèce est cultivée dans des conditions optimales pour sa croissance.

### Exemples. -

**Exemple 1.-** Composition selon la présente invention utilisée pour traiter biologiquement l'eau d'un aquarium.

Nous avons préparé une composition comprenant *Bacillus subtilis* (4 x 10^10 bactéries/gramme, avec 1 gramme (gr) dans 800 grammes de mytho), et Paracoccus pantatrophus (1,4 x 10^11 bactéries/gramme, avec 1 gramme dans 2800 grammes de mytho, qui est un mélange minéral comprenant une source de calcium et de carbonate de calcium, comme par exemple le produit CALCIMER qui est une matière fertilisante homologuée, AMM N°1120009).

Nous avons ensuite rempli d'eau un aquarium de 15L dans lequel nous avons déposé dans le fond de l'aquarium des carrelages et céramique apte à accueillir un biofilm bactérien.

Nous avons ajouté la composition comprenant 1,5 gr de *Bacillus subtilis* et 1,5 gr de Paracoccus pantatrophus dans l'aquarium de manière quotidienne pendant 5 jours : du premier jour (jour 1) au cinquième jour (jour 5), et également au jour 12.

Nous avons également ajouté quotidiennement dans l'aquarium 0,3 gr de nourriture pour poisson pendant toute la durée de l'expérience : du jour 1 au jour 18. La température est restée substantiellement constante à 20°C durant toute la durée de l'expérience : du jour 1 au jour 18.

Nous avons ensuite mesuré chaque jour, du jour 1 au jour 18, différents paramètres physico-chimiques à l'aide d'un capteur photomètre (spin Touch) de manière à déterminer la dureté, l'alcalinité, le pH, le phosphate, l'ammoniaque, le nitrite et le nitrate. La turbidité, le potentiel redox ainsi que la température ont également été mesurés. La turbidité a été mesurée à l'aide d'un turbidimètre, comme par exemple un turbidimètre de type Turbiquant 1100 (Mercks).

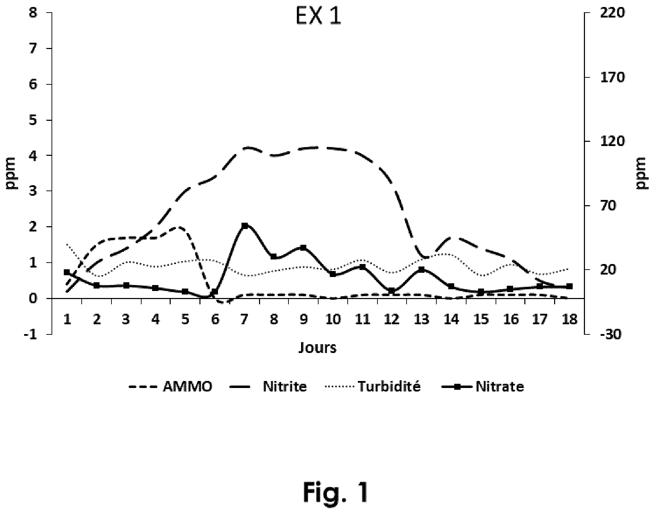

La figure 1 montre l'évolution temporelle de la quantité d'ammoniaque, de nitrite, de nitrate ainsi que la turbidité dans l'aquarium lorsque l'aquarium a été traité par la composition selon l'exemple 1 (EX 1).

**Exemple 2.-** Première composition comparative utilisée pour traiter biologiquement l'eau d'un aquarium.

L'Exemple 1 a été reproduit excepté que la composition ne comprend pas 1,5 gr de *Bacillus subtilis* et 1,5 gr de Paracoccus pantatrophus, mais comprend 1,5 gr d'un cocktail bactérien (Bactogen) correspondant au cocktail bactérien décrit à l'Exemple 7 de la demande de brevet EP3712116.

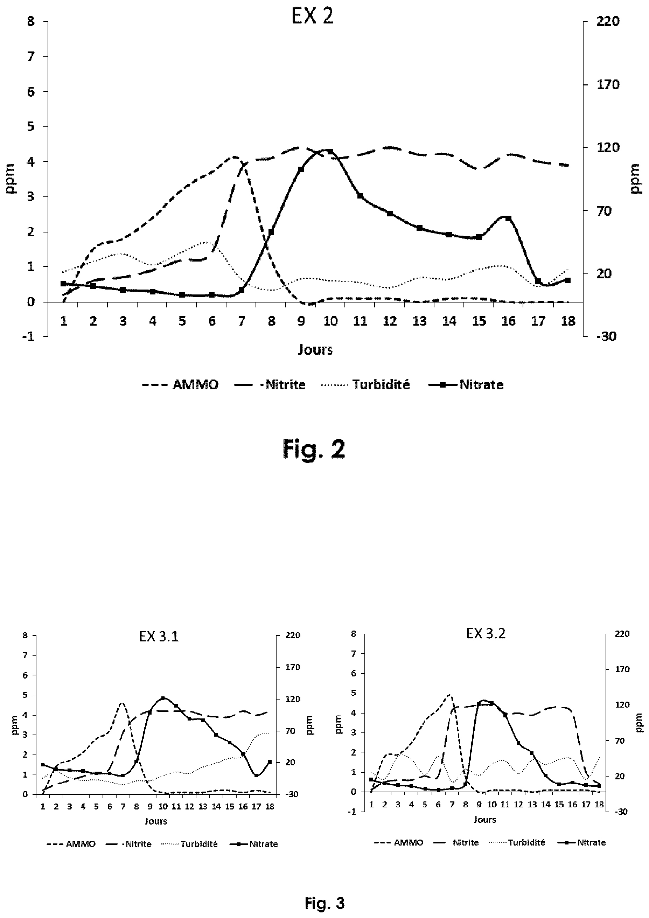

La figure 2 montre l'évolution temporelle de la quantité d'ammoniaque, de nitrite, de nitrate ainsi que la turbidité dans l'aquarium lorsque l'aquarium a été traité par la composition selon l'exemple 2 (EX 2).

**Exemple 3.-** Deuxième composition comparative utilisée pour traiter biologiquement l'eau d'un aquarium.

L'Exemple 1 a été reproduit excepté que la composition comprend uniquement 1,5 gr (EX 3.1) ou 6 gr (EX 3.2) de Paracoccus pantatrophus.

La figure 3 montre l'évolution temporelle de la quantité d'ammoniaque, de nitrite, de nitrate ainsi que la turbidité dans l'aquarium lorsque l'aquarium a été traité par la composition selon l'exemple 3 (1,5 gr et 6 gr de Paracoccus pantatrophus).

**Exemple 4.-** Troisième composition comparative utilisée pour traiter biologiquement l'eau d'un aquarium.

L'Exemple 1 a été reproduit excepté que la composition ne comprend pas 1,5 gr de *Bacillus subtilis* et 1,5 gr de Paracoccus pantatrophus, mais comprend 1,5 gr d'un cocktail bactérien (Bactogen) correspondant au cocktail bactérien décrit à l'Exemple 7 de la demande de brevet EP3712116 et 1,5 gr (EX 4.1) ou 6 gr (EX 4.2) de Paracoccus pantatrophus.

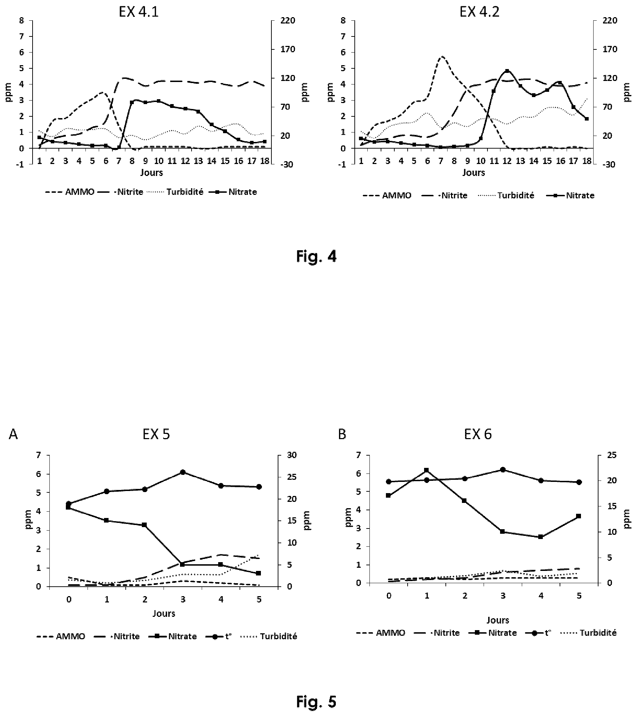

La figure 4 montre l'évolution temporelle de la quantité d'ammoniaque, de nitrite, de nitrate ainsi que la turbidité dans l'aquarium lorsque l'aquarium a été traité par la composition selon l'exemple 4 :
- 1,5 gr de Paracoccus pantatrophus + 1,5 gr de cocktail bactérien (Bactogen) (EX 4.1), et
- 6 gr de Paracoccus pantatrophus + 1,5 gr de cocktail bactérien (Bactogen) (EX 4.2).

**Exemple 5.-** Composition selon la présente invention utilisée pour traiter biologiquement l'eau d'une piscine.

De manière similaire à l'Exemple 1, nous avons préparé une composition comprenant *Bacillus subtilis* (4 x 10^10 bactéries/gramme, avec 1 gramme (gr) dans 800 grammes de mytho), et Paracoccus pantatrophus (1,4 x 10^11 bactéries/gramme, avec 1 gramme dans 2800 grammes de mytho).

Nous avons ajouté au début de l'expérience (jour 1), dans un piscine contenant 6 m³ d'eau, la composition à raison de 5 gr de *Bacillus subtilis* et 5 gr de Paracoccus pantatrophus par m³ d'eau, soit 30 gr de *Bacillus subtilis* et 30 gr de Paracoccus pantatrophus.

Nous avons également ajouté 120 gr de nourriture pour poisson dans la piscine au début de l'expérience (jour 1).

Nous avons ensuite mesuré chaque jour, du jour 1 au jour 5, différents paramètres physico-chimiques à l'aide d'un capteur photomètre (spin Touch) de manière à déterminer la dureté, l'alcalinité, le pH, le phosphate, l'ammoniaque, le nitrite et le nitrate. La turbidité, le potentiel redox ainsi que la température ont également été mesurées.

La figure 5A montre l'évolution temporelle de la quantité d'ammoniaque, de nitrite, de nitrate ainsi que la turbidité dans la piscine lorsque celle-ci a été traité par la composition selon l'exemple 5 (EX 5).

**Exemple 6.-** Première composition comparative utilisée pour traiter biologiquement l'eau d'un d'une piscine.

L'Exemple 5 a été reproduit excepté que la composition ajoutée à la piscine ne comprend pas 5 gr de *Bacillus subtilis* et 5 gr de Paracoccus pantatrophus par m³ d'eau, mais comprend 5 gr par m³ d'eau (soit 30 gr) d'un cocktail bactérien (Bactogen) correspondant au cocktail bactérien décrit à l'Exemple 7 de la demande de brevet EP3712116.

La figure 5B montre l'évolution temporelle de la quantité d'ammoniaque, de nitrite, de nitrate ainsi que la turbidité dans la piscine lorsque celle-ci a été traité par la composition selon l'exemple 6 (EX 6).

### Résultats.-

### 1. Traitement biologique d'une eau à petite échelle par la composition selon l'invention (EX 1) et par des compositions comparatives (EX 2 à EX 4.2)

La Figure 1 montre une complémentarité parfaite entre *Bacillus subtilis* et Paracoccus pantotrophus de la composition selon l'invention pour accomplir les différentes étapes du cycle de l'azote (voir EX 1). En effet, le pic de nitrites est très similaire quant à son amplitude par rapport aux conditions des exemples comparatifs (voir EX 2, EX 3.1, EX 3.2, EX 4.1 et EX 4.2). Par contre, ce pic de nitrites est beaucoup plus limité dans le temps avec un retour proche de zéro dès le 13^{ème} jour.

De plus, avec la composition selon l'invention (Ex 1), le pic de nitrates est très faible, voire inexistant par rapport aux autres conditions. Ceci suggère que *Bacillus subtilis* et Paracoccus pantotrophus se sont chacune installée dans une voie métabolique complémentaire à l'autre, parvenant de la sorte à une dégradation particulièrement efficace de la matière organique et de l'élimination des résidus.

### 2. Traitement biologique d'une eau à grande échelle par la composition selon l'invention (EX 5) et par une composition comparative (EX 6)

Les résultats obtenus à petite échelle ont été reproduit à plus grande échelle dans laquelle on peut observer une diminution du pic de nitrites et une réduction plus rapide du pic de nitrates en présence de la composition selon la présente invention (EX 5) par rapport à une composition comparative comprenant un cocktail bactérien (EX 6).
Il est bien entendu que la présente invention n'est en aucune façon limitée aux formes de réalisations décrites ci-dessus et que bien des modifications peuvent y être apportées sans sortir du cadre des revendications annexées.

## Revendications

1. Composition pour le traitement biologique des systèmes aquatiques, comprenant un agent actif bactérien, ledit agent actif bactérien comprenant des bactéries du genre *Bacillus* et Paracoccus, **caractérisée en ce que** la quantité des bactéries du genre *Bacillus* et Paracoccus dans ledit agent actif bactérien est supérieure ou égale à 80% en poids par rapport au poids total dudit agent actif bactérien dans la composition.

2. Composition selon la revendication 1, dans laquelle ledit agent actif bactérien consiste en des bactéries du genre *Bacillus* et Paracoccus.

3. Composition selon l'une quelconque des revendications précédentes, dans laquelle lesdites bactéries du genre *Bacillus* et Paracoccus présente un rapport en poids Bacillus/Paracoccus compris entre 1:10 et 10:1, préférentiellement compris entre 1:5 et 5:1.

4. Composition selon l'une quelconque des revendications précédentes, dans laquelle lesdites bactéries du genre *Bacillus* comprennent *Bacillus subtilis.*

5. Composition selon la revendication précédente, dans laquelle *Bacillus subtilis* a un poids égal ou supérieur à 80% du poids total desdites bactéries du genre Bacillus.

6. Composition bactérienne selon l'une quelconque des revendications précédentes, dans laquelle lesdites bactéries du genre Paracoccus comprennent Paracoccus pantotrophus.

7. Composition selon la revendication précédente, dans laquelle Paracoccus pantotrophus a un poids égal ou supérieur à 80% du poids total desdites bactéries du genre Paracoccus.

8. Composition selon l'une quelconque des revendications précédentes, caractérisée en ce ledit agent actif bactérien comprend *Bacillus subtilis* et Paracoccus pantotrophus, et en ce que la quantité de *Bacillus subtilis* et Paracoccus pantotrophus dans ledit agent actif bactérien est supérieure ou égale à 80% en poids par rapport au poids total dudit agent actif bactérien dans la composition.

9. Composition selon l'une quelconque des revendications précédentes, comprenant en outre un agent enzymatique.

10. Composition selon l'une quelconque des revendications précédentes, comprenant en outre un agent minéral.

11. Composition selon l'une quelconque des revendications précédentes, comprenant en outre un agent de conservation.

12. Utilisation de la composition selon l'une quelconque des revendications précédentes 1 à 11 pour traiter biologiquement un système aquatique.

13. Procédé de préparation de la composition selon l'une quelconque des revendications précédentes 1 à 11, dans lequel chacune des bactéries dudit agent actif bactérien est fermentée, optionnellement séchée et récoltée individuellement.

14. Filtre biologique pour des bassins d'eaux récréatives comprenant, en mode opérationnel, un substrat colonisé par lesdites bactéries dudit agent actif bactérien de ladite composition selon l'une quelconque des revendications 1 à 11.

15. Système aquatique comprenant le filtre biologique selon la revendication précédente.
